# EUROPÄISCHE PATENTSCHRIFT

(11) **EP 3 651 685 B1**
(45) Veröffentlichungstag und Bekanntmachung des Hinweises auf die Patenterteilung: **01.09.2021**
(21) Anmeldenummer: 18742737.2
(22) Anmeldetag: 10.07.2018
(51) Int. Cl.: A61C 8/02, A61F 2/28, A61L 27/58, A61F 2/30

(54) **KNOCHENAUGMENTATIONSSTÜCK UND BAUSATZ AUS KNOCHENAUGMENTATIONSSTÜCK MIT EINGESETZTEM (ZAHN-) IMPLANTAT**
BONE AUGMENTATION PIECE, AND KIT CONSISTING OF A BONE AUGMENTATION PIECE WITH AN INSERTED (DENTAL) IMPLANT
PIÈCE D'AUGMENTATION OSSEUSE ET KIT COMPOSÉ D'UNE PIÈCE D'AUGMENTATION OSSEUSE COMPORTANT UN IMPLANT (DENTAIRE) INSÉRÉ

(30) Priorität: 10.07.2017 DE 102017115404
(43) Veröffentlichungstag der Anmeldung: 20.05.2020
(73) Patentinhaber: Karl Leibinger Medizintechnik GmbH & Co. KG, 78570 Mühlheim (DE)
(72) Erfinder: RÜCKER, Martin, 8032 Zürich (CH); ESSIG, Harald, 8044 Zürich (CH); REINAUER, Frank, 78576 Emmingen-Liptingen (DE); AKSU, Adem, 78054 Villingen-Schwenningen (DE)
(74) Vertreter: Winter, Brandl - Partnerschaft mbB
(86) Internationale Anmeldenummer: PCT/EP2018/068664
(87) Internationale Veröffentlichungsnummer: WO 2019/011915

(56) Entgegenhaltungen:
- US-A- 6 077 076
- US-A1- 2006 052 873
- US-A1- 2017 014 550
- US-B1- 6 214 049

## Beschreibung

Die Erfindung betrifft ein Knochenaugmentationsstück gemäß Anspruch 1 zum Auffüllen einer Fehlstelle, wie einem Loch, einem Schlitz oder einer Lücke, in einem Knochen, wie einem Ober- oder Unterkieferknochen, aber außerhalb eines Dysgnathie-Anwendungsbereiches.

Aus dem Stand der Technik sind bereits zahlreiche Knochenimplantate bekannt, bisher aber keine, die als Knochenaugmentationsstück eine Fehlstelle in einem Knochen auffüllen. Üblicherweise werden Platten außenseitig des Knochens aufgebracht, um das Loch bzw. die Fehlstelle in ihrer Position zu fixieren und nachfolgend wird in die Fehlstelle dann Knochenfüllmaterial, wie Granulat eingegeben. Allerdings weist dieses Granulat immer den Nachteil auf, dass es per se nicht formstabil ist. Es müssen die einzelnen Granulatbestandteile mühsam in die Fehlstelle verbracht werden, dort verdichtet werden, um dann erst im Laufe der Zeit zu verfestigen.

Das Dokument US2017014550 A offenbart ein implantierbares Knochenaugmentationsstück zur osteogenen Regeneration mit einem biologisch abbaubaren Polymermaterial.

Es soll aber gerade ein schnellerer Heilungserfolg und eine bessere Handhabbarkeit für den Operateur erreicht werden. Auch soll ein schnell einwachsendes, kostengünstiges und einen guten operativen Erfolg gewährleistendes Knochenimplantat zur Verfügung gestellt werden.

Bei Verlust von Knochen im Bereich des zahntragenden Kieferkammes, bspw. im Rahmen von Unfällen, Resektionen bei gut- oder bösartigen Tumoren, Entzündungen, und weiteren Indikationen, ist meist eine Rekonstruktion des knöchernen Kieferkammes (Augmentation) und die spätere Insertion von dentalen Implantaten indiziert. Ein übliches Vorgehen stellt dabei die autogene Knochentransplantation dar. Hierfür wird der Knochen regional (z.B. im Mittelgesicht / im nicht zahntragenden Anteil des Unterkiefers), insbesondere aus der Beckenschaufel, entnommen. Zur Vermeidung eines darauf folgenden Entnahmedefektes und einer Morbidität im Spenderareal werden zunehmend alloplastische Granulate zur Augmentation des Kieferkammes eingesetzt. Diese Granulate sind allerdings nicht formstabil und müssen durch zusätzliche Membranen vor einem unerwünschten Einwachsen von Bindegewebe geschützt werden. Zuletzt wurden 3D-Titanmeshes entwickelt, die diesen Nachteil der fehlenden Formstabilität adressieren, jedoch vor einer geplanten dentalen Implantation wieder vollständig entfernt werden müssen.

Die autogene Knochentransplantation hat jedoch die Nachteile der Entnahmemorbidität im Spenderareal, eine aufwändige Konturierungsnotwendigkeit an die Defektsituation und Limitation in der lokoregionären Verfügbarkeit des Knochens. Alloplastische Granulate sind leider nicht formstabil und benötigen Membrane, da sie keine Membraneigenschaft per se besitzen und ermöglichen keine integrierte 3D-Planung. 3D-Titanmeshes sind leider nicht resorbierbar, haben auch keine Membraneigenschaft und müssen vor einer Implantation notwendigerweise entfernt werden. Gerade eine Vielzahl von Eingriffen ist jedoch unerwünscht.

Es ist daher die Aufgabe der vorliegenden Erfindung, diese Nachteile zu beseitigen.

Diese Aufgabe wird erfindungsgemäß dadurch gelöst, dass das Knochenaugmentationsstück zum Ausfüllen / Auffüllen einer Fehlstelle mit einem Grundkörper versehen ist, der angepasst ist, (möglichst) spaltfrei in die Fehlstelle eingesetzt zu werden, wobei der massive oder makro- / mikroporöse Grundkörper eine fachwerkartige Struktur aus Stegen / Strängen, die selber auch massiv oder makro- / mikroporös ausbildbar sind, und Hohlräumen besitzt, etwa um formstabil zu sein, und dass der Grundkörper biodegradierbare metallische Werkstoffe und/oder resorbierbare Polymere oder Keramikwerkstoffe enthält oder daraus aufgebaut ist. Diese initiale Formstabilität hilft nämlich beim Einbringen des Knochenaugmentationsstückes.

Außerdem wird durch die fachwerkartige Struktur aus einem Material, das sich biologisch abbaut, zum einen das Einwachsen von Knochenmaterial in das Implantat ein schneller Knochenaufbau unterstützt und zum anderen eine nahezu ursprüngliche bzw. natürliche Struktur hergestellt, da sich das Implantat abbaut und somit nur das Knochenmaterial im Körper verbleibt. Ebenso wird durch die gitterartige Struktur ein Abbau des körperfremden Materials beschleunigt, da ein großer Oberflächenkontakt zwischen dem Knochenmaterial und dem Implantat realisiert ist.

Vorteilhafte Ausführungsformen sind in den Unteransprüchen beansprucht und werden nachfolgend näher erläutert.

So ist es von Vorteil, wenn sich mehrere Stege kreuzen und die Stege am Kreuzungspunkt miteinander verbunden sind, vorzugsweise dort einstückig / einmaterialig, so dass sie am Kreuzungspunkt ein integrales Bauteil ausformend verbunden sind.

Es ist zweckmäßig, wenn die Stege eine Gitterstruktur bilden. Dabei bilden sich Maschen. Diese Maschen sollen dann alle oder überwiegend die (annähernd oder exakt) gleiche Fläche besitzen. Dadurch kann das Einwachsen genau gesteuert bzw. vorausgesagt werden. Das Durchwachsen der Gitterstruktur mit aufbauendem Knochenmaterial ist dann besonders gleichmäßig, was einen guten operativen Erfolg nach sich zieht.

Für die Fertigung ist es von Vorteil, wenn die Maschen eine Dreieckform, insbesondere die eines gleichseitigen oder gleichschenkligen Dreiecks besitzen oder die Maschen eine Rechteckform, insbesondere die eines gleichseitigen Rechtecks / Quadrats besitzen. Die Anordnung der Stege / Stränge ist dann besonders gut vorbestimmbar, was der Fertigung zuträglich ist.

Die initiale Formstabilität wird besonders effizient festlegbar / auslegbar, wenn die Stege, bspw. alle Stege oder die überwiegende Anzahl der Stege geometrisch und/oder bezüglich der Materialeigenschaften gleich beschaffen sind.

Dabei ist es von Vorteil, wenn die Stege linear / gerade oder wellig oder kurvenförmig ausgebildet sind. Auch abschnittsweise linear mit einem vorgeschalteten und/oder nachgeschalteten Übergang in eine wellige / kurvenartige Form ist von Vorteil. Auch hat es sich bewährt, wenn bei Ausbildung einer Kurvenform immer die gleiche Krümmung vorhanden ist. Dabei soll hierbei nicht unerwähnt bleiben, dass eine über die Länge gesehen zunehmende Krümmung oder alternativ abnehmende Krümmung Vorteile aufweisen kann. Auch eine Verdrillung der Stege ist denkbar.

Ein vorteilhaftes Ausführungsbeispiel ist auch dadurch gekennzeichnet, dass die Stege und Maschen eine Wandung definieren, die nach Art einer Tasche ausgebildet ist. So kann bspw. eine Öffnung an dem Knochenaugmentationsstück / dem Knochenimplantat vorhanden sein, bspw. an der Ober- oder Unterseite, um Füllungen aufzunehmen, wie bspw. Knochenschlamm / Knochenspäne.

Von Vorteil ist es, wenn der Grundkörper Löcher / Kavitäten von 10 µm bis 700 µm aufweist. Bei Vorhalten einer Porosität haben sich Lufteinschlüsse mit mittleren Erstreckungen von 10 µm bis 200 µm bewährt. Die Tasche ist vorgesehen, mit allografischem Material, wie Blut oder Knochenspänen befüllt zu werden.

Die Stege und Maschen sind außenseitig von einem vollständig geschlossenen oder teilgeschlossenen Rand umgeben. Der Rand kann dabei eine umlaufend gleiche Dicke aufweisen oder variieren. Das Vorhandensein eines Randes erleichtert die Handhabbarkeit. Letztlich kann bei der Implantatsplanung ein Implantatskanal modifiziert werden, bspw. im Bereich seiner Wandung und als Bohrschablone benutzt werden. Bei der Implantatsplanung kann auch eine Überkorrektur angelegt sein, nämlich derart, dass der Grundkörper über die Fehlstelle hinaus ragt, insbesondere auch im eingesetzten Zustand, um dem Rechnung zu tragen, dass mehr vom Grundkörper während der Heilung abgebaut wird, als neues Knochenmaterial aufgebaut wird. Dies dient dem Ziel eine ästhetisch ansprechende Lösung zu erreichen. So werden Dellen oder Vertiefungen, welche lediglich von Haut bedeckt sind, aber von außen visuell wahrnehmbar sind, vermieden.

Als besonders vorteilhaft hat es sich auch herausgestellt, wenn die Dicke der Stege zwischen einem Viertel bis einem Zehntel der Länge der Stege zwischen zwei Kreuzungspunkten beträgt und/oder der Querschnitt der Stege polygonartig oder kreisartig oder elliptisch ausgebildet ist. Besonders dreieckige, viereckige und kreisrunde Querschnitte haben sich bewährt. Bei den dreieckigen Querschnitten hat sich die Form eines gleichseitigen Dreiecks besonders bewährt.

Um eine Endversorgung des Patienten besonders gut zu gewährleisten, ist es von Vorteil, wenn in dem Grundkörper wenigstens ein (Durchgang- / Sack-) Implantataufnahmeloch zur Aufnahme eines Implantats, wie eines Zahnimplantats vorhanden ist. So kann bspw. schon ein Zahnimplantat in dem Grundkörper angebracht werden und dieser Bausatz dann während einer Operation eingesetzt werden. Natürlich betrifft die Erfindung auch das Einsetzen des Knochenaugmentationsstückes in den Knochen bzw. das Einsetzen des Bausatzes in/an den Knochen.

Von Vorteil ist es, wenn eine das Implantataufnahmeloch definierende Lochwand eine geschlossene / lochfreie Oberfläche besitzt. Ein guter Halt bspw. eines Zahnimplantates im Grundkörper ist dann die Folge.

Wenn der Grundkörper wenigstens ein Knochenbefestigungsloch besitzt, nämlich zum Aufnehmen eines Befestigungsmittels, wie einer Knochenschraube, für eine Festlegung am Knochen, so kann schon initial eine gute Verbindung zwischen dem Knochenaugmentationsstück / dem Knochenimplantat und dem Knochen erreicht werden. Das nachfolgende Einwachsen verstärkt diese initiale Festigkeit noch.

Es sei nicht unerwähnt, dass der Grundkörper vorteilhafter Weise aus einem (einzigen) Material aufgebaut sein kann und soll, wie einem biodegradierbaren Werkstoff wie Magnesium, einer Magnesiumlegierung, Eisen, einer Eisenlegierung, Barium oder Strontium, oder einem resorbierbaren Polymer, einem keramischen Material oder deren Kompositen, etwa PDLLA, PDLA, PLA, PGA, Chitosanfasern / -partikel, HAP, CaCo₃, α-/β-TCP, Hydroxylapatit und/oder biphasischem Kalziumphosphat (BCP). Auch Mischungen der genannten Materialien sind natürlich denkbar.

Die Erfindung betrifft letztlich auch den Bausatz aus einem Knochenaugmentationsstück der erfindungsgemäßen Art mit darin eingesetztem Implantat, wie einem Zahnimplantat. Die Erfindung betrifft auch die Verbindung des Knochenaugmentationsstücks der erfindungsgemäßen Art als Bohrschablone.

Auch betrifft die Erfindung ein medizinisches Verfahren zum Einsetzen des Knochenaugmentationsstückes der erfindungsgemäßen Art an/in einen Knochen, bspw. eines Säugetiers, insbesondere eines Menschen.

Mit anderen Worten wird also nun eine formstabile An- und Auflagerung mit alloplastischem Material (Eigenmaterial) ermöglicht. Dabei wird eine bedarfsadäquate Vorgehensweise ermöglicht, da ausgehend von der geeigneten dentalen Implantatsposition die Form und Größe des 3D-Konstruktes schon initial festgelegt werden kann. Es werden Verbesserungen im Contouring- und Containment-Bereich erreicht. Ein Resorptionsschutz durch Membraneigenschaften der Außenkontur wird vorgehalten. Dies vermeidet eine Bindegewebeeinsprossung. Es werden Verletzungen anatomischer Nachbarstrukturen vermieden, insbesondere von Nachbarzähnen, Nachbarzahnwurzeln und, was besonders kritisch ist, des nervus alveolaris inferior. Es ist nämlich möglich vorgeplante Fixationspunkte am Restknochen zu nutzen, also preoperativ die Operation exakt durchzuplanen.

Es werden massive und/oder poröse, geometrisch komplexe und patientenspezifisch gefertigte Individualimplantate aus Knochenersatzmaterialien, wie HA, α-TCP, β-TCP, BCP, Magnesium oder Magnesium-Kalzium-Zink-Legierungen eingesetzt und vorhergestellt. Eine kurzzeitige Herstellung der porösen, geometrisch komplexen und patientenspezifischen Individualimplantat-Vorrichtung aus Knochenersatzmaterial, wie HA, α-TCP, β-TCP, BCP, Magnesium und MgCaZn wird ermöglicht. Eine Festigkeitssteigerung der gefertigten Implantate ist durch eine Wärmesteigerung machbar. Auch ist die Herstellung von massiven und/oder porösen geometrisch komplexen und patientenspezifisch angefertigten Individualimplantaten aus Knochenmaterialien als resorbierbares Knochenhohlraum-Füllerkonstrukt zur Knochenregeneration die Folge. Eine kurzzeitige /kurzfristige Herstellung der porösen, geometrisch komplexen und patientenspezifischen Individualimplantate aus Knochenersatzmaterialien ist machbar. Das Implantat kann aus einem oder mehreren, z.B. zwei Materialien bestehen.

Alle Materialien sind in Pulverform, Granulatform und flüssig bis zähflüssig vorliegend einsetzbar, wobei unterschiedliche Mengen und Stoffzusammensetzungen miteinander vermischbar sind.

Abschließend sei erwähnt, dass das Knochenaugmentationsstück Vorsprünge, Ohren, Erweiterungen, Laschen oder Plattenerweiterungsabschnitte außenseitig, insbesondere distal abstehend vorhanden sein Können, um eine Überdeckung mit dem angrenzenden Knochenmaterial einzugehen. In diesen Vorsprüngen können auch Löcher vorangelegt sein, um Knochenschrauben aufzunehmen. Ein plattenfreies Anbinden des Knochenaugmentationsstückes am Knochen kann dann bewirkt werden.

Die Erfindung wird nachfolgend mit Hilfe einer Zeichnung näher erläutert. Es zeigen:
- Fig. 1: eine Seitenansicht eines Knochenaugmentationsstückes mit einem im Grundkörper vorhandenen umlaufenden Rand, innerhalb dessen zahlreiche äquidistant zueinander angeordnete Stege eine Gitterstruktur ausbilden,
- Fig. 2: eine perspektivische Ansicht von der dem Knochen zugewandten Seite des Knochenaugmentationsstückes aus Fig. 1 mit ausgebildeter Tasche zur Befüllung mit Knochenschlamm / -spänen an der Implantatsunterseite und oben vorhandener Öffnung,
- Fig. 3: eine Gitterstruktur eines nicht beanspruchten Augmentationsstückes ohne umlaufenden Rand,
- Fig. 4: eine vergleichbare Gitterstruktur zu der aus Fig. 3, jedoch mit umlaufendem Rand,
- Fig. 5: ein schematischer Aufbau einer Tasche, wie sie in Fig. 2 dargestellt ist,
- Fig. 6: der Einsatz des Knochenaugmentationsstückes als Bohrschablone, und
- Fig. 7: eine vorgegebene Überkorrektur mittels des Knochenaugmentationsstückes sowie
- Fig. 8: den eingesetzten Zustand des in den Fign. 1 und 2 dargestellten Knochenaugmentationsstückes in einem Patienten-Unterkiefer.

Die Figuren sind lediglich schematischer Natur. Die gleichen Elemente sind mit denselben Bezugszeichen versehen. Merkmale der einzelnen Ausführungsbeispiele können untereinander ausgetauscht werden.

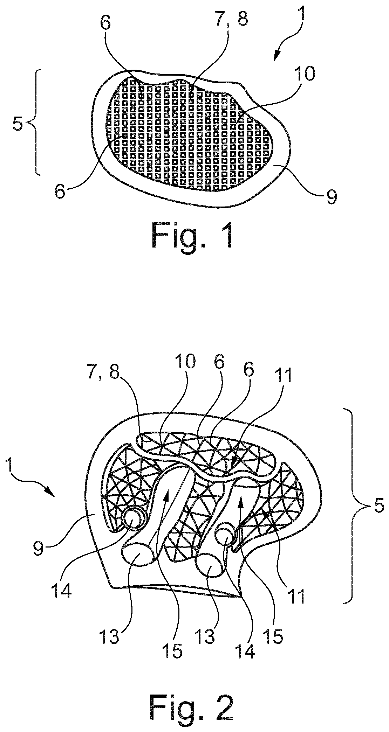

In der Fig. 1 ist ein erfindungsgemäßes Knochenaugmentationsstück 1 im Sinne der Erfindung dargestellt. Es ist ein Knochenimplantat. Insbesondere ist es ein Knochenimplantat, das zur Verwendung im Unterkiefer angepasst ist. Es könnte, mit leichten Adaptionen, auch im Oberkieferbereich eingesetzt werden.

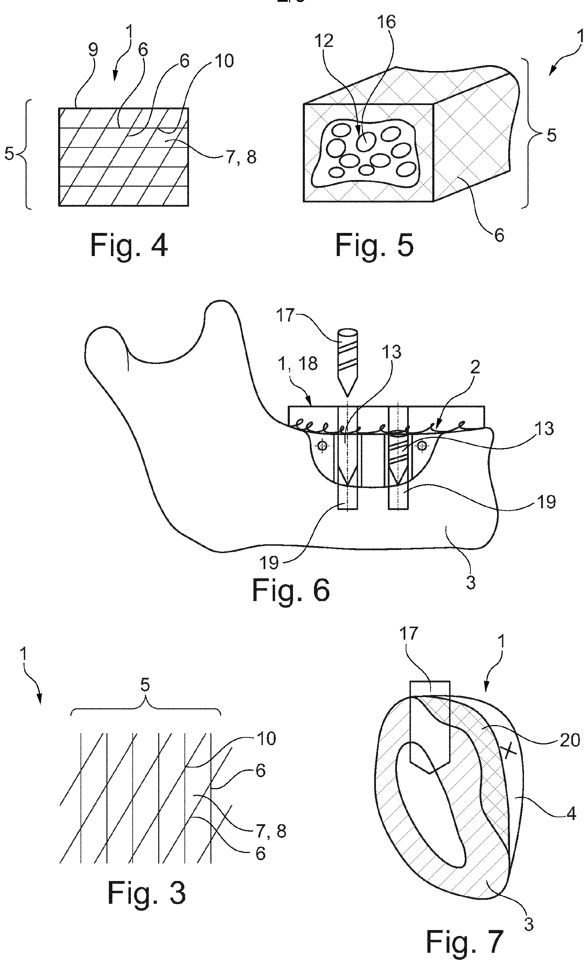

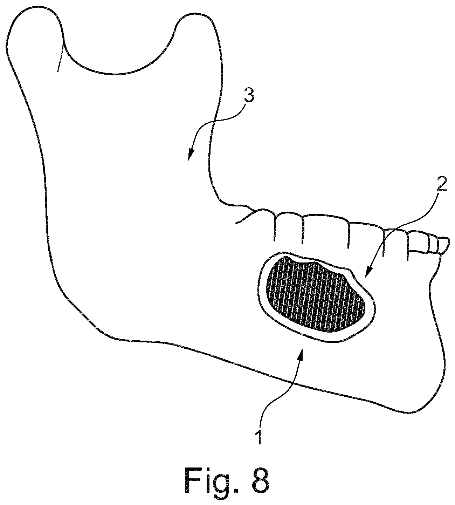

Unter Vorgriff auf Fig. 8 sei bereits erläutert, dass das Knochenaugmentationsstück 1 eine Fehlstelle 2 in dem Knochen 3 ausfüllt. Es ist in die Fehlstelle 2, die nach Art eines Sacklockes oder Durchgangsloches ausgebildet sein kann, schlitzfrei / spaltfrei eingesetzt. Im vorliegenden Fall ist es bündig mit der Oberfläche des Knochens 3. Es ist zum stufenlosen Übergehen in den Knochen 3, also Anliegen der Oberflächen des Knochenaugmentationsstückes einerseits und des Knochens 3 andererseits nämlich auf der Innenseite und auf der Außenseite konfiguriert. Es kann aber auch (entweder an einer oder beiden Flächen) ein kleiner Überstand vorhanden sein, wie er in Fig. 7 dargestellt ist und mit dem Bezugszeichen 4 referenziert ist.

Zurückkommend auf Fig. 1 ist erkennbar, dass das Knochenaugmentationsstück 1 einen Grundkörper 5 aus einer Vielzahl von sich kreuzenden Stegen 6 besitzt. Die Stege 6 können auch als Stränge bezeichnet werden. Zwischen den Stegen 6 gibt es eine Vielzahl von Hohlräumen 7, die Maschen 8 definieren.

Die Stege 6 kreuzen sich fachwerkartig bspw. rechtwinklig, um eine Gitterstruktur zu erzeugen.

Wie in dem alternativen Ausführungsbeispiel der Fig. 2 erkennbar ist, sind die Maschen 8 nicht zwingenderweise rechteckförmig, sondern können auch dreieckförmig ausgebildet sein. Der Grundkörper 5 weist neben den Stegen 6 und den durch sie gebildeten Maschen 8 auch einen geschlossenen Rand 9 auf. Der Rand 9 ist im vorliegenden Fall mehr als fünf Mal so dick wie ein Steg 6. Von Vorteil ist es, wenn der Rand 9 elf Mal so dick (+/- 10 bis 20 %) wie ein Steg 6 ist.

Exemplarisch sind in den Fign. 1 und 2 Kreuzungspunkte 10 referenziert.

Die Stege 6 und die durch sie gebildeten Maschen 8, umfassend den Rand 9, wenn dieser vorhanden ist, definieren eine Wandung 11 oder mehrere Wandungen 11, mittels der eine Tasche 12 ausgebildet ist.

Es fällt auf, dass zwei Implantataufnahmelöcher 13 vorhanden sind. Zusätzlich gibt es auch noch Knochenbefestigungslöcher 14. Während in dem Implantataufnahmeloch 13 ein weiteres Implantat, wie ein Zahnimplantat, eingesetzt wird und am Knochen 3 und/oder dem Grundkörper 5 befestigt wird, ist im Knochenbefestigungsloch 14 ein Befestigungsmittel, wie eine Schraube, ein Splint oder ein Bolzen einbringbar, der den Grundkörper 5 am Knochen 3 festlegt. Das Knochenbefestigungsloch 14 kann zumindest teilweise innerhalb des Implantataufnahmeloches 13 angeordnet sein oder sogar vollständig. Natürlich kann es auch außerhalb davon angeordnet sein. Besonders bevorzugt ist es, wenn das Knochenbefestigungsloch 14 eine das Implantataufnahmeloch 13 definierende Lochwand 15 teilweise durchdringt.

In den Fign. 3 und 4 sind unterschiedliche Steganordnungen für einen Grundkörper 5 angedeutet, wobei einmal ein Rand 9 vorhanden ist (siehe Fig. 4) und einmal jener fehlt (Fig. 3).

In der Fig. 5 ist ein Knochenaugmentationsstück 1 mit einem Grundkörper 5, der nach Art einer Tasche 12 ausgebildet, ist dargestellt. In die Tasche 12 sind dabei Knochenspäne bzw. Knochenschlamm 16 eingefüllt. Hier können auch Blutprodukte hinzugefügt sein.

In der Fig. 3 ist das Eingesetzt sein eines Knochenaugmentationsstückes 1 in eine Fehlstelle 2 eines Knochens 3 dargestellt. Dabei ist zu erkennen, dass die Implantataufnahmelöcher 13 zur Zwangsrichtungsvorgabe für einen Bohrer 17 eingesetzt sind, sodass das Knochenaugmentationsstück 1 als Bohrschablone 18 wirkt. Die in den Knochen weiterführenden Versenkungen 19 werden dann zum Durchdrungen werden durch ein nicht dargestelltes Zahnimplantat verwendet.

In Fig. 7 ist der Unterkieferknochen, als Beispiel eines Knochens 3 im Querschnitt dargestellt und ein eingesetztes erfindungsgemäßes Knochenaugmentationsstück 1 teilweise im Schnitt wiedergegeben. Dabei gibt es einen Überstand 4. Dieser Überstand 4 wird genauso, wie der Rest des Knochenaugmentationsstückes 1 während der Heilung vom Körper des Patienten abgebaut, wobei jedoch nur im geschnitten dargestellten Bereich, der auch als Verbleibbereich 20 definiert werden kann, knocheneigenes Material nach der Heilung vorhanden ist, sodass ein ästhetisch ansprechendes Ergebnis erreicht ist.

### Bezugszeichenliste

- 1: Knochenaugmentationsstück
- 2: Fehlstelle
- 3: Knochen
- 4: Überstand
- 5: Grundkörper
- 6: Steg
- 7: Hohlraum
- 8: Masche
- 9: Rand
- 10: Kreuzungspunkt
- 11: Wandung
- 12: Tasche
- 13: Implantataufnahmeloch
- 14: Knochenbefestigungsloch
- 15: Lochwand
- 16: Knochenschlamm / Knochenspan
- 17: Bohrer
- 18: Bohrschablone
- 19: Versenkung
- 20: Verbleibbereich

## Patentansprüche

1. Knochenaugmentationsstück (1) zum Ausfüllen einer Fehlstelle (2) in einem Knochen (3), mit einem Grundkörper (5), der angepasst ist, spaltfrei in die Fehlstelle (2) eingesetzt zu werden, **dadurch gekennzeichnet, dass** der Grundkörper (5) eine fachwerkartige Struktur aus Stegen (6) und Hohlräumen (7), die Maschen (8) definieren, besitzt, biodegradierbare metallische Werkstoffe und/oder resorbierbare Polymere oder Keramikwerkstoffe enthält oder daraus aufgebaut ist, und die Stege (6) und Maschen (8) außenseitig von einem geschlossenen Rand (9) umgeben sind, wobei der Rand (9) mehr als fünfmal so dick wie ein Steg (6) ist.

2. Knochenaugmentationsstück (1) nach Anspruch 1, **dadurch gekennzeichnet, dass** sich mehrere Stege (6) kreuzen und am Kreuzungspunkt (10) miteinander verbunden sind.

3. Knochenaugmentationsstück (1) nach Anspruch 1 oder 2, **dadurch gekennzeichnet, dass** die Stege (6) eine Gitterstruktur bilden, in der die überwiegende Anzahl der Maschen (8) eine gleiche Fläche besitzen.

4. Knochenaugmentationsstück (1) nach Anspruch 3, **dadurch gekennzeichnet, dass** die Maschen (8) eine Dreieckform oder eine Rechteckform besitzen.

5. Knochenaugmentationsstück (1) nach einem der Ansprüche 1 bis 4, **dadurch gekennzeichnet, dass** die Stege (6) bzgl. ihrer Geometrie und/oder bzgl. der Materialeigenschaften gleich beschaffen sind.

6. Knochenaugmentationsstück (1) nach einem der Ansprüche 1 bis 5, **dadurch gekennzeichnet, dass** die Stege (6) linear / gerade oder wellig oder kurvenförmig ausgebildet sind.

7. Knochenaugmentationsstück (1) nach einem der Ansprüche 1 bis 6, **dadurch gekennzeichnet, dass** die Stege (6) und Maschen (8) eine Wandung (11) definieren, die nach Art einer Tasche (12) ausgebildet ist.

8. Knochenaugmentationsstück (1) nach einem der Ansprüche 2 bis 7, wenn vom Anspruch 2 abhängig, **dadurch gekennzeichnet, dass** die Dicke der Stege (6) zwischen einem Viertel bis einem Zehntel der Länge der Stege (6) zwischen zwei Kreuzungspunkten (10) beträgt .

9. Bausatz aus einem Knochenaugmentationsstück (1) nach einem der vorhergehenden Ansprüche mit darin eingesetztem Implantat.

## Claims

1. A bone augmentation piece (1) for filling out a defective area (2) in a bone (3), comprising a main part (5) which is adapted to be inserted into the defective area (2) without a gap, **characterized in that** the main part (5) comprises a truss-type structure made of webs (6) and cavities (7) which define meshes (8), contains or is made up from biodegradable metallic materials and/or resorbable polymers or ceramic materials, and the webs (6) and meshes (8) are surrounded on the outside by a closed rim (9), the rim (9) having more than five times the thickness of a web (6).

2. The bone augmentation piece (1) according to claim 1, **characterized in that** a plurality of webs (6) are intersecting and are interconnected at the intersection point (10).

3. The bone augmentation piece (1) according to claim 1 or 2, **characterized in that** the webs (6) form a grid structure in which the majority of the meshes (8) has an equal surface area.

4. The bone augmentation piece (1) according to claim 3, **characterized in that** the meshes (8) have a triangular shape or a rectangular shape.

5. The bone augmentation piece (1) according to one of the claims 1 to 4, **characterized in that** the webs (6) are identical as to their geometry and/or as to the material properties.

6. The bone augmentation piece (1) according to one of the claims 1 to 5, **characterized in that** the webs (6) are linear/straight or corrugated or curved.

7. The bone augmentation piece (1) according to one of the claims 1 to 6, **characterized in that** the webs (6) and meshes (8) define a wall (11) designed in the form of a pocket (12).

8. The bone augmentation piece (1) according to one of the claims 2 to 7, if dependent on claim 2, **characterized in that** the thickness of the webs (6) ranges from one quarter to one tenth of the length of the webs (6) between two intersection points (10).

9. A kit consisting of a bone augmentation piece (1) according to one of the preceding claims comprising an implant inserted into it.

## Revendications

1. Pièce d'augmentation osseuse (1) pour combler un défaut (2) dans un os (3) comprenant un corps de base (5) qui est formé de manière à être inséré sans jeu dans le défaut (2), **caractérisé en ce que** le corps de base (5) possède une structure de type treillis constituée d'éléments de liaison (6) et de cavités (7) qui définissent des mailles (8), **en ce qu'**il contient des matériaux métalliques biodégradables et/ou des polymères résorbables ou des matériaux céramiques ou est constitué de ceux-ci, et les éléments de liaison (6) et les mailles (8) sont entourés sur la face externe d'un bord fermé (9), dans laquelle le bord (9) est plus que cinq fois aussi épais qu'un élément de liaison (6).

2. Pièce d'augmentation osseuse (1) selon la revendication 1, **caractérisée en ce que** plusieurs éléments de liaison (6) se croisent et sont reliés les uns aux autres au niveau d'un point de croisement (10).

3. Pièce d'augmentation osseuse (1) selon les revendications 1 ou 2, **caractérisée en ce que** les éléments de liaison (6) forment une structure en treillis dans laquelle la majorité des mailles (8) possèdent une surface identique.

4. Pièce d'augmentation osseuse (1) selon la revendication 3, **caractérisée en ce que** les mailles (8) possèdent une forme triangulaire ou une forme rectangulaire.

5. Pièce d'augmentation osseuse (1) selon l'une quelconque des revendications 1 à 4, **caractérisée en ce que** les éléments de liaison (6), du point de vue de leur géométrie et/ou du point de vue des propriétés des matériaux, sont de même nature.

6. Pièce d'augmentation osseuse (1) selon l'une quelconque des revendications 1 à 5, **caractérisée en ce que** les éléments de liaison (6) sont configurés linéaires/droits ou ondulés ou incurvés.

7. Pièce d'augmentation osseuse (1) selon l'une quelconque des revendications 1 à 6, **caractérisée en ce que** les éléments de liaison (6) et les mailles (8) définissent une paroi (11) qui est configurée à la manière d'une poche (12).

8. Pièce d'augmentation osseuse (1) selon l'une quelconque des revendications 2 à 7, si dépendantes de la revendication 2, **caractérisée en ce que** l'épaisseur des éléments de liaison (6) est comprise entre un quart à un dixième de la longueur des éléments de liaison (6) entre deux points de croisement (10).

9. Kit composé d'une pièce d'augmentation osseuse (1) selon l'une quelconque des revendications précédentes comportant un implant inséré à l'intérieur de celui-ci.
